(19)

Europäisches Patentamt

European Patent Office

Office européen des brevets

(11) **EP 4 379 038 A2**

(12) **EUROPEAN PATENT APPLICATION**

(43) Date of publication:
**05.06.2024 Bulletin 2024/23**

(51) International Patent Classification (IPC):
**C12M 1/00** *(2006.01)*

(21) Application number: **23213306.6**

(22) Date of filing: **30.11.2023**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **02.12.2022 DE 102022132083**

(71) Applicant: **Horia Hulubei National Institute for R & D in
Physics and Nuclear Engineering (IFIN-HH)
077125 Magurele (RO)**

(72) Inventors:
• **Jurca, Marius
63486 Bruchköbel (DE)**
• **Spohr, Klaus, Dr.
Paisley, Scotland, PA2 0TX (GB)**

(74) Representative: **Ostertag & Partner Patentanwälte mbB
Azenbergstraße 35
70174 Stuttgart (DE)**

(54) **METHOD AND DEVICE FOR PORATING AND LOADING CELLS, ESPECIALLY IMMUNOCOMPETENT CELLS**

(57) The invention provides a method for porating cells for loading the cells with a substance, preferably with nuclides for radiotherapy in a porating device, wherein the cells to be porated are provided in a liquid together with the substance. The sonoporation or laser poration is performed in a reactor under real-time-monitoring. Moreover, the invention provides a device for porating cells, in particular by sonoporation or laser porating, and for loading cells, in particular immunocompetent cells, in particular $\gamma\delta$ T cells. The device comprises (a) an inlet opening and an outlet opening for a cell solution with cells to be porated and at least one substance to be taken up by the cells as a load and preferably microbubbles, wherein the poration arrangement has a collecting unit (99) arranged at the inlet opening for collecting the cell solution and a reactor in flow connection with the collecting unit (99), and wherein inside the reactor the cell membranes of the cells may be porated and the load may be uptaken by the cells.

Fig. 5

EP 4 379 038 A2

**Description**

The invention relates to a method and a device for porating, in particular for sonoporation or laser poration, and for loading cells, in particular immunocompetent cells, in particular γδ T cells.

Cell therapy, in particular T-cell therapy, is a novel and proven method for the treatment of genetically modified cells, especially viral genetically modified or altered cells, including most recently Covid-19, or patient-specific malignant tumor cells.

Recent studies highlight the unique potential for new γδ CAR-T cell-based treatments (Mirzaei et al. (2016): "Prospects for chimeric antigen receptor (CAR) T cells: A potential game changer for adoptive T cell cancer immunotherapy"). These special cells have a specific T-cell receptor (TCR) on their surface. In their natural form, γδ T cells make up 2% to 10% of peripheral blood T lymphocytes. Among others, the techniques developed for αβ-T cell therapies can be applied.

A decisive advantage of γδ T cells over αβ T cells is the innate tumor recognition capability of γδ T cells, which enables the targeted killing of cancerous tissue. If genetically modified γδ CAR-T cells are used, in which the specificity towards patient-specific genetically modified cells has been increased by a chimeric anti-gene receptor (CAR), the side effects of treatment can be kept to a minimum.

The particular cancer selectivity of genetically modified γδ CAR-T cells is based on the ability of the chimeric antigen cell receptor (CAR) introduced into the cells by genetic engineering to recognize the isopentetyl pyrophosphate (IPP) antigen located on the surface of the cancer cell due to a disturbed isoprenoid signaling pathway in the body of the affected cell (Zeng, Jieming et al. (May 2019): "Derivation of mimetic T cells endowed with cancer recognition receptors from reprogrammed T cell").

In response to the recognition of a cancer cell, the attacking γδ CAR-T cells produce inflammatory cytotoxic substances, which they inject into the degenerated, i.e. malignant, cell via the so-called "kiss-of-death" mechanism (Rozenbaum, Meir et al. (July 2020): "Gamma-Delta CAR-T Cells Show CAR-Directed and Independent Activity Against Leukemia"). γδ T cells have been shown *in vitro* and clinical experiments to be very versatile and have already shown promising results in clinical studies and are becoming increasingly established in the treatment of leukemia, neuroblastoma, various carcinomas and, most recently, Covid-19 (Caron et al. (Mar. 2021): "How to Train Your Dragon: Harnessing Gamma Delta T Cells Antiviral Functions and Trained Immunity in a Pandemic Era").

As the field of immunotherapy moves beyond the repeated iteration of established methods to more creative solutions, γδ CAR-T cells offer the potential to advance established cancer treatments and break new ground in oncology. These new methods are broad-spectrum and have a much more favorable safety profile than conventional therapies, as the surrounding healthy cell tissue is preserved during treatment due to the specificity of the γδ CAR-T cells.

In conventional therapy with αβ CAR-T cells, for example, two signals that stimulate the T cells are triggered by activating the chimeric antigen receptor (CAR) on the outside of the T cell. A disadvantage of αβ-CARs is that they can also bind to healthy cells, which leads to the death of healthy cells after activation of the T cells.

This phenomenon is referred to as "off-tumor toxicity" and causes strong side effects, such as those observed with classic chemotherapy. γδ CAR-T cells differ from other T cells in that they have a specific γδ T cell receptor (TCR) that only binds to antigens which are characteristic of impaired unhealthy cells. The TCR serves as a safety barrier against activation of the T cells in healthy cells. A genetically modified γδ CAR-T cell, which only has one co-stimulatory domain, only binds to one damaged cell. This reduces side effects, as healthy cells are not attacked.

In order to obtain sufficient cell material, work is currently being carried out on allogeneic cell pools for which γδ T cells are taken from healthy donors, multiplied and distributed worldwide via a distribution system.

The potential of γδ T cells is further enhanced by the recently developed method, which makes it possible to switch from the individual autologous therapy (see US 9,855,298 B2) to allogeneic therapy.

The allogeneic use of γδ T cells reduces graft-versus-host disease (GvHD), even if this initially appears to be a contradiction in terms. This is possible because the current γδ CAR-T cell technology allows to overcome the restriction triggered by the major histocompatibility complex (MHC). Therefore, the risk of GvHD minimized (Handgretinger et al. (Mar. 2018): "The potential role of T cells after allogeneic HCT for leukemia"). In addition, various studies have recorded an overall favorable effect of high γδ CAR-T cell immune reconstitution, e.g. after hematopoietic stem cell transplantation (HSCT). In accompanying clinical studies, patients with an increased number of γδ CAR-T cells had a significantly higher overall survival rate and undetectable acute GvHD manifestations. In addition, the likelihood of transplant rejection in patients with severe viral infections such as HIV, influenza and Covid-19 is low, and corresponding advanced clinical trials have been underway since early 2022.

It is also known that solid tumors are subject to further mutations over time, which also change the structure of the externally accessible receptors, which can currently only be recognized after a histological or histo-pathological examination, followed by more detailed examination methods.

Therapy approaches with immunocompetent cells known from the state of the art can be found, for example, in WO 2022241151 A2, WO 2022238962 A1, WO 2022241036 A1, WO 2022147014 A2 and WO 2020109953 A1.

Further therapeutic approaches can be found in WO 2019 181018 A1, WO 9817342 A2, CN 115215851 A, CN 115232129 A, FR 3120630 A1, CN 112675442 A and JP 2016159107 A.

For example, for the form of therapy described above, but also in general for cell therapy it may be beneficial to load cells with a substance. This substance may, for example, be a substance that is to be transported from the cells to certain target cells. Nanoparticles and/or cytotoxic substances are particularly suitable here if the target cells are to be attacked. In the case of the form of therapy described above, such substances can drastically improve the success of the treatment.

Approaches known from the prior art for loading cells use the principle of increasing the permeability of the cell membranes by means of poration of the same, for example electroporation, sonoporation or laserporation. Corresponding devices are known, for example, from JP 2018099059 A, US 2010196983 A1, US 2022062611 A1 and WO 2007028984 A1.

However, it remains difficult to porate and load the very sensitive immunocompetent cells like $\gamma\delta$ T cells or $\gamma\delta$ CAR-T cells, because the loading efficiency is very low. In general, there are too few of the treated cells that ultimately carry the desired load in relation to the effort required to porate and load the cells and then heal them before they can divide again.

In view of these disadvantages of prior art loading approaches the present invention has the object to improve the loading of cells with a substance, in particular of immunocompetent cells or other very sensitive cells, for example $\gamma\delta$ T cells, in order to improve the loading efficiency.

This object is solved by a method for porating cells for loading the cells with a substance as it is described in claim 1. Preferred embodiments of the claimed method are outlined in the dependent claims. Furthermore, this object is solved by a device for porating and for loading cells according to the further independent claim and, in preferred embodiments, in the further dependent claims.

The method according to the invention is provided for porating cells, preferably immunocompetent cells, in order to load the cells with a substance. Preferably said substance or substances are nuclides for radiotherapy. The porating is performed in a porating device, wherein the cells to be porated are provided in a liquid together with the substance, preferably in the presence of microbubbles. The membranes of the cells are permeabilized by sonoporation or laser poration. As a main point of the inventive method, the sonoporation is performed in a reactor under real-time monitoring.

The real-time monitoring of the poration and/or loading process allows for an especially effective loading of the cells, because it is possible to control the number and vitality of the cells and especially to control the loading of the cells. In preferred embodiments the cells to be porated and loaded are very sensitive cells, like immunocompetent cells. Such cells are extremely sensitive in view of stress conditions like mechanical stress and/or stress induced by the porating conditions. Therefore, it is advantageous to apply mild conditions for porating and loading. However, the mild conditions may result in only poor loading of the cells. The porating and loading of the cells therefore represents a balancing act between efficient loading and maintaining vital cells that are capable of further cell proliferation. This balancing act can be supported in a very favourable way by real-time monitoring.

The substance(s) to be loaded by the cells can be different cytotoxic substances or nanoparticles. Especially preferred are nuclides for radiotherapy or other therapeutic approaches. Preferably, the nuclides are at least one stable isotope and/or at least one radioisotope from the following group of stable isotopes or radioisotopes: $^{10}B$, $^{11}B$, $^{nat}B$, $^{157}Gd$, $^{99m}Tc$, $^{177}Lu$, $^{18}F$, and $^{211}At$ or mixtures thereof. $^{10}B$ and/or $^{157}Gd$ are especially preferred.

In especially preferred embodiments the nuclides are intended to be activated later by supportive radiotherapy, e.g. in connection with boron neutron capture therapy (BNCT).

In especially preferred embodiments the real-time monitoring is microscopic and/or radiometric monitoring. Microscopic monitoring enables a very efficient control of the vitality of the cells. A radiometric monitoring is especially useful if nuclides for radiotherapy are the substances which are to be absorbed by the cells. By such monitoring measures it is possible to monitor the state of membrane permeabilization and the cell healing process after poration. Moreover, it is possible to determine the relative number of successfully loaded cells and to evaluate these factors for further control and regulation of the process.

Preferably, the sonoporation or laser poration is performed in a capillary section of the porating device. It is particularly preferred if the capillary section has a flattened form. The flatted form is especially useful in view of the monitoring, e.g., a microscopic monitoring. In especially preferred embodiments the capillary section is made of an optically transparent material, like glass.

In especially preferred embodiments of the inventive method the cells are incubated in an incubation section of the porating device after sonoporation or laser poration. Advantageously the incubation in the incubation section of the porating device is performed with gentle turbulence of the liquid.

By the additional incubation step after the poration itself the efficiency of the incorporation respectively loading of the substance into the cells can be considerably increased.

Moreover, it is preferred that the incubation in the incubation section of the porating device is performed with application of an electric or electromagnetic field into the incubation section of the porating device in order to control and possibly prolong the permeabilization of the cell membranes. In this context it is especially preferred to apply electric or electromagnetic fields which are below the forces which are applied for poration, because the applied electric or electromagnetic fields during incubation shall only control the permeabilization and shall not porate the cells any more. Preferably, the electric or electromagnetic fields applied during incubation are modulated according to a predetermined scheme or are modulated in dependence of a feedback monitoring.

Moreover, in preferred embodiments the liquid with the cells from the incubation section is recirculated into the reactor of the porating device, in which repeated poration takes place. This is an especially advantageous measure to increase the loading capacity of the described method, particularly in combination with the real-time monitoring and a control of the process.

Further features and advantages of the inventive method arise from the following description in connection with an advantageous device for performing the inventive method.

The invention further comprises a device for porating, in particular for sonoporation or laser poration, and for loading cells, in particular immunocompetent cells, in particular $\gamma\delta$ T cells. The devices comprises:

a. an inlet opening and an outlet opening for a cell solution with cells to be porated and at least one substance to be taken up by the cells as a load and preferably microbubbles, wherein a poration arrangement extends between the inlet opening and the outlet opening, wherein the poration arrangement has a collecting unit arranged at the inlet opening for collecting the cell solution and a reactor in flow connection with the collecting unit, and wherein inside the reactor the cell membranes of the cells may be porated and the load may be uptaken by the cells, and

b. a poration unit arranged on the reactor, by means of which energy can be applied to the cell solution in such a way that the cell membranes of at least some of the cells are porated respectively permeabilized.

As a main feature of the device according to the invention the reactor comprises a capillary section in which the cells can be porated under real-time monitoring.

The invention is based on the finding that the efficiency of the porating and loading process is improved if the poration takes place in a very small reaction volume. Especially the generation of pores within the cell membranes and the proportion of cells that have been effectively loaded with the substance is improved compared to larger volumes. Moreover, the efficiency of the porating and loading process is improved by the real-time monitoring which allows the number of cells and the vitality of the cells as well as the loading of the cells to be controlled.

In especially preferred embodiments the poration arrangement comprises microscopic and/or radiometric means for real-time monitoring. Preferably, the poration arrangement comprises one or more corresponding control units like microscopic and/or radiometric control units. The control units may be arranged at different positions within the poration arrangement, especially in the area of the reactor and/or at further positions within the poration arrangement as described in further detail below.

In especially preferred embodiments the means for real-time monitoring are based on spectroscopy, diffraction and confocal measurement technology.

Preferably, the capillary section has an internal diameter or cross-section of 0.01 mm to 1 mm.

In especially preferred embodiments of the inventive device the capillary section has a flattened form. The flatted form is especially useful in view of the monitoring, e.g., a microscopic monitoring. In especially preferred embodiments the capillary section provides a flat and large surface area.

Preferably, an inlet of the capillary section opens into the collection unit and/or an outlet of the capillary section opens into an incubation section downstream of the reactor which will be explained in more detail below.

It is advantageous if the capillary section of the reactor extends over essentially the entire length of the reactor, i.e. the reactor is designed as a capillary.

It is advantageous if the capillary section provides a linear or meandering flow path for the cell solution.

There is also an advantage if the poration unit is a first poration unit and a second poration unit is arranged on the reactor, the first poration unit being arranged on a first side of the reactor and the second poration unit being arranged on a second side of the reactor opposite the first side. That means, in preferred embodiments the reactor comprises two poration units, preferably opposite to each other.

It is also advantageous if the poration unit or, if applicable, at least one of the poration units is a sound transducer, in particular an ultrasonic transducer, by means of which a sound pressure, in particular an ultrasonic pressure, can be generated on the basis of an electrical signal and can be applied to the cell solution in the reactor.

Alternatively or additionally, at least one of the poration units may be a laser. By means of a laser beam, in particular a top-hat laser beam, e.g. a top-hat intensity profile can be generated and applied to the cell solution in the reactor in order to permeabilize the cell membranes.

Preferably, the collection unit and the capillary section of the reactor are arranged such that the cell solution, when added to the collection unit, is sucked into the capillary section of the reactor by a capillary force, thereby generating in particular a self-sustaining flow through the poration arrangement.

In especially preferred embodiment the poration arrangement comprises a tubular incubation section between the reactor and the outlet opening. Preferably, the incubation section is located downstream of the reactor and is therefore arranged outside the reactor respectively outside the region (capillary section) within which the poration is carried out.

Within the tubular incubation section, the porated cells can be incubated with the substance to be loaded for the purpose of enriching loaded cells. The fraction of cells that are effectively loaded with the substance when leaving the device can be significantly increased if the cells are incubated in the incubation section of the device for a certain period of time after the actual poration.

The incubation step enables the cells to continue to absorb the substance in the cell solution while they take measures to close the pores over a certain period of time, for example by transporting material for the cell membranes and/or the cytoskeleton to the areas affected by lesions in the cell membranes.

The duration of incubation is preferably 30 to 60 minutes.

Advantageously, the cell solution in the incubation section can be heated by at least one heating element arranged on or in the poration arrangement. Preferably, the cell solution in the incubation section is heated and incubated at a constant temperature, in particular at constant 37° C.

In especially preferred embodiments of the device according to the invention, the incubation section comprises at least one turbulence structure, by which a turbulence can be generated within the cell solution when the cell solution flows through the incubation section.

Preferably, the turbulence is a gentle movement of the cell solution so that the sensitive cells are not subjected to excessive mechanical stress.

In especially preferred embodiments, the incubation section comprises a plurality of turbulence structures which together may define a meandering flow path through the incubation section.

Preferably, the at least turbulence structure is designed as a baffle or chicane within the incubation section. Alternatively or additionally, the tubular incubation section may be designed to have the form of a U-shaped course.

The generated turbulence is preferably unpressurised. The turbulence structures, e.g. baffles, therefore preferably have a non-hydrodynamic shape that disrupts and does not support the laminar flow of the cell mixture.

Furthermore, in especially preferred embodiments, the device comprises at least one control unit in the area of the incubation section which allows for real-time monitoring in this area. Preferably, the at least one control unit is arranged at the incubation section, by means of which the quality and/or the quantity of the loaded cells can be checked and controlled. Preferably, the control unit enables radiometric and/or microscopic control, preferably chromatically-confocal. In especially preferred embodiments the control unit is set up to detect ionizing particle radiation.

In especially preferred embodiments a return line is arranged between the incubation section and the outlet opening, within which a pump unit is preferably arranged. Preferably, the return line ends in the collection unit. By means of the return line the cells may be recirculated to the reactor in order to increase the amount of cells which have been effectively loaded with the substance. Preferably, the recirculation is under control by means of real-time monitoring.

In especially preferred embodiments the poration arrangement comprises filter means, e.g. a centrifugation filter, in order to separate fractions with loaded and fractions with non-loaded cells. The fraction with non-loaded cells may be recirculated into the reactor via the return line.

In especially preferred embodiments the device comprises a generator unit. By means of the generator unit a stationary or non-stationary electric or electromagnetic field can be applied to the cell solution in the reactor and/or in the incubation sector to control permeabilization of the cell membranes.

Preferably, the poration arrangement comprises transparent metal electrodes which may be placed on opposite walls of the poration arrangement, preferably in the section of the poration units and/or in the incubation section. Such transparent metal electrodes are especially useful for applying an electric or electromagnetic field to the corresponding sections of the poration arrangement in order to control the permeabilization of the cell membranes.

In preferred embodiments the poration arrangement comprises a flow adjustment element, in particular a pump wheel or a turbine. The flow adjustment element is set up to bring about a constant flow of the cell solution through the poration arrangement.

According to a further aspect of the invention, the invention comprises a method for porating, in particular for sonoporating or laser porating, and for loading cells, in particular immunocompetent cells, in particular $\gamma\delta$ T cells. The method is characterized by being carried out using a device having some or all of the features mentioned above in connection with the description of the device.

Further features and advantages of the invention result from the following description of further embodiments in connection with the drawings. The individual features here can each be implemented individually or in combination with each other.

BRIEF DESCRIPTION OF THE DRAWINGS

In the figures it is shown:

Figure 1    a schematic representation of a $\gamma\delta$ CAR-T cell;

Figure 2    a schematic representation of microbubbles carrying nanoparticles;

Figures 3a) to d)    a schematic and theoretical representation of a charging process using sonoporation;

Figure 4    a schematic representation of examples of the $\gamma\delta$ CAR-T cells which are hereinafter referred to as "T-ninja cells";

Figures 5a) and b)    a schematic representation of a device for porating, in this case sonoporation, and for loading cells, as well as a schematic enlargement of the cell solution in a reactor;

Figure 6    a schematic representation of a porating unit for laser porating and for loading cells; and

Figure 7    a schematic representation of a uniform distribution of the loaded substances to daughter cells after division of the T-ninja cells.

DESCRIPTION OF EXEMPLARY EMBODIMENTS

In the following, embodiments of the invention are explained in more detail with reference to the figures. The figures show embodiments of the invention, and the following explanations relate to these embodiments. It is clear to an expert that the explanations of the specific embodiments of the invention are in no way restrictive of the general idea of the invention.

An effective expansion and distribution method for allogeneic transfer is known, for example, from WO 2016005752 A1, and has led to positive results in clinical studies. The procedure described there begins with the initial collection of $\gamma\delta$ T cells taken from a healthy first donor or, for example, umbilical cord blood. An enrichment to 99% of $\gamma\delta$ T cells is then achieved before multiplication by several orders of magnitude in this process.

Quantities of $2.5\times10^{10}$ cells can be obtained from a single donor within 10 to 18 days. The optimum time for a loading with substances is agreed upon by clinicians after thorough testing with the clinics in which the expansion and enrichment process of the $\gamma\delta$ CAR-T cells is carried out.

An international network of dedicated allogeneic supply chains in Europe, the USA and Asia, which includes cryogenic facilities, will enable the storage of $\gamma\delta$ CAR-T cells, which can be used for therapies for a period of up to two to three weeks after their exponential multiplication.

The loading of cells, specifically $\gamma\delta$ CAR-T cells, with a substance, in particular with nanoparticles and/or a cytotoxic substance like nuclides for radiotherapy, using a method and device for porating and loading cells according to the invention represents an advantageous extension of loading processes known from the prior art.

In therapies with $\gamma\delta$ CAR-T cells, for example, their unique cancer recognition ability is used, whereby the $\gamma\delta$ CAR-T cells can be adjusted so that only certain cancer cells are attacked. With the support of cytokines (e.g. IL-2 or IL-12), the cancer cells are then specifically attacked, so that cells loaded by means of the described method and device can minimize the side effects that can occur which are caused by an attack on the patient's healthy cells.

Patient-specific genetically modified cells in the form of, for example, malignant tumor cells in the patient are initially attacked by the T-cell's own cytotoxic substances and can then be further attacked by the release of the loaded nanoparticles or the loaded cytotoxic substance. The nanoparticles can, for example, be activated at a later stage by supportive radiotherapy.

Nanoparticle technologies have a significant impact on the development of therapeutic and diagnostic agents (theranostics). At the interface between treatment and diagnosis, interest in clinically effective nanoparticles is growing. Most of these new developments see nanoparticles as carriers of drugs or contrast agents. Various contrast agents have been used in clinical studies to observe their interactions with the specific disease. Most commonly, optically active small molecules, metals and metal oxides, ultrasound contrast agents, and radionuclides are used. Theranostics is of great importance for active substances that act on molecular biomarkers of disease and will, therefore, contribute to personalized medicine for efficient treatments.

The focus of these nanoparticles is on specific isotopes that are relevant to cancer treatment, with $^{10}$B, which is used in boron neutron capture therapy (BNCT) (Malouff et al. (Feb. 2021): "Boron Neutron Capture Therapy: A Review of Clinical Applications") is the most important example of the nanoparticles described here, whereby other nanoparticles and/or a cytotoxic substance are also used for the purpose of monitoring and controlling the density and distribution of the $\gamma\delta$ CAR-T-cell loading at a desired time, even before irradiation, for example with neutron radiation, separately or together.

The nanoparticles can be any nanoparticles that are currently relevant in nuclear medicine. At present, as already mentioned, $^{10}B$ is primarily used for BNCT. Gadolinium $^{157}Gd$ is very interesting for a similar procedure, here called GdNCT in reference to BNCT, as this isotope has the highest neutron capture cross-section in nature, so that much less irradiation is required for GdNCT. Therefore very sensitive regions of the body can also be treated. Both isotopes are not naturally radioactive, but are only stimulated to decay when they are activated by an external neutron source, for example an accelerator or a nuclear reactor. The maximum effective cross-section for triggering the necessary decays is in the epithermal to thermal energy range of the neutrons, corresponding to a kinetic energy of around 0.025 eV.

The BNCT and the GdNCT utilize the fission reaction that occurs when non-radioactive $^{10}B$ or $^{157}Gd$ are irradiated with thermal or epithermal neutrons $n_{th}$. The tissue of the body is transparent to such neutrons so that they can penetrate the tissue unhindered. However, the epithermal neutrons lose a small amount of energy when they penetrate tissue, so that they become thermal neutrons - they are "thermalized".

$$^{10}B + n_{th} \rightarrow {}^4He + {}^7Li + 2.8 \text{ MeV} \qquad (1a)$$

$$^{157}Gd + n_{th} \rightarrow [{}^{158}Gd] \rightarrow {}^{158}Gd + \gamma + 7.94 \text{ MeV} \qquad (1b)$$

According to formula (1a), a high-energy $\alpha$-particle and a $^7Li$-ion are generated, both of which lose a kinetic energy determined by the Q value of the reaction through linear energy transfer (LET) as they pass through the cell. $^7Li$ and the lighter $^4He$ reach a maximum flight range of ~ 4 $\mu$m for $^7Li$ and ~ 9 $\mu$m for $^4He$ during fission. Since these lengths correspond to the diameter of a human cell, the lethality of the capture reaction is only limited to cells in the immediate vicinity of the boron nanoparticles. The success of BNCT therefore depends on the selective release of sufficiently high amounts of $^{10}B$ to the tumor itself, whereby only small amounts are localized in the surrounding healthy tissue. The same applies to GdNCT and formula (1b). This is one of the core applications for a therapy with the cells loaded according to the invention.

The above-mentioned naturally occurring stable isotopes have the largest effective radii, which are given in the relevant technical literature as 3,837 barns for $^{10}B$ and 254,000 barns for $^{157}Gd$.

Currently, in the best case, the boron load of a patient-specific genetically modified cell, in particular a cancer cell, can be increased only three- to fourfold compared to the neighboring healthy surrounding cells. In accordance with the invention this enhancement factor is improved by four to six orders of magnitude with a simultaneous reduction in side effects. The limitation of previous methods is especially disadvantageous for BNCT because normal tissue tolerates the flow of slow neutrons due to the relatively low nuclear capture reactions that occur with hydrogen and nitrogen. In comparison, the unavoidable effects associated with thermal and epithermal neutron irradiation are not as significant. Thus, the inventive method and device for porating and loading cells will enable BNCT to become an effective cancer treatment procedure.

Radiation doses to which healthy tissue is exposed may be minimized by the porated and loaded cells according to the invention in combination with the BNCT. A single fission of a $^{10}B$ isotope is sufficient to disrupt a patient-specific genetically modified cell, in particular a tumor-like malignant cell.

The isotope $^{10}B$ makes up around 20% of the low-cost $^{nat}B$. $^{nat}B$ nanoparticles contain a large proportion of $^{11}B$, which is also stable and can be used for radiotherapy. $^{11}B$ nanoparticles have a modest effective cross-section for proton radiation with an energy of approx. 600 keV (1 barn), i.e. immunocompetent cells in the form of $\gamma\delta$ CAR-T cells loaded with them can also be used for proton radiotherapy.

In addition to the non-radioactive isotopes mentioned above, other radioisotopes and/or isomers can be considered for cancer theranostics, for example, e.g. $^{99m}Tc$, $^{177}Lu$, $^{18}F$ and $^{21}At$ as well as all other isotopes and/or isomers which are currently of medical relevance for cancer or other treatments that target patient-specific genetically modified cells that have at least potentially pathogenic potential. In general, these are either commonly used radiologic isotopes or those currently used in advanced stages of clinical trials where they may be used to treat a variety of malignancies.

For the isotopes described in the previous paragraph, the radioactive activity differs as a function of time A(t), which is referred to here for the described biological activity of the $\gamma\delta$ CAR-T cells.

The activity A(t) describes the decay of a radioactive sample per second and is defined by

$$A(t) = \lambda \times N(t) \qquad (2)$$

where $N(t) = N_0 \times \exp(-\lambda \times t)$ is the number of isotopes in the sample, $\lambda$ is the decay constant for a particular isotope and $N_0$ is the amount of isotopes at an arbitrarily chosen reference time $t_0$.

Physicians can calculate the time frame and the quantity of nanoparticles of the above-mentioned isotopes with comparatively high precision. In contrast to A(t), biological activation does not follow such a simple exponential rule. Instead, it depends on a variety of controllable external factors, such as cell growth, survival rate and cryopreservation for later "revival".

The dose rates required to activate a therapeutic amount of radioactive decay are calculated based on continuous or pulsed sources, which are currently available at specially expanded nuclear reactor sites or radiofrequency (RF) powered accelerators, or Linear Tandem Accelerators.

Furthermore, modern laser-powered accelerators will soon be available for clinical use, which will deliver comparatively high temporal beam doses and can therefore minimize the total time for patient irradiation.

The activities A(t) of the theranostic sample are adjusted accordingly for each treatment to determine the optimal dose rate:

$$D = dE/dm = (1/\rho) \; dE/dV \qquad (3)$$

where dE describes the energy released within a mass segment dm in the patient's body with a specific density of $\rho$, which is distributed over a volume element of dV.

For precise calculations, the heterogeneous structures of the tissue would have to be grouped into inhomogeneity volumes Di before they are summed up to D to derive a treatment plan. The total dose rate D is given in microgray per hour [$\mu$Gy/h] and converted into the equivalent dose rate, given in Sievert per hour [Sv/h].

**Process steps in detail**

Step 1 Loading of cells in the form of $\gamma\delta$ CAR-T cells with a substance in the form of nanoparticles and/or a cytotoxic substance by sonoporation

In the following, reference is made to the figures and the reference signs shown therein, whereby the figures only show specific embodiments of the invention. Reference signs are generally only assigned once in each the following. However, it will be readily apparent that the same reference signs identify the same structures in the figures, unless another reference sign has been explicitly assigned.

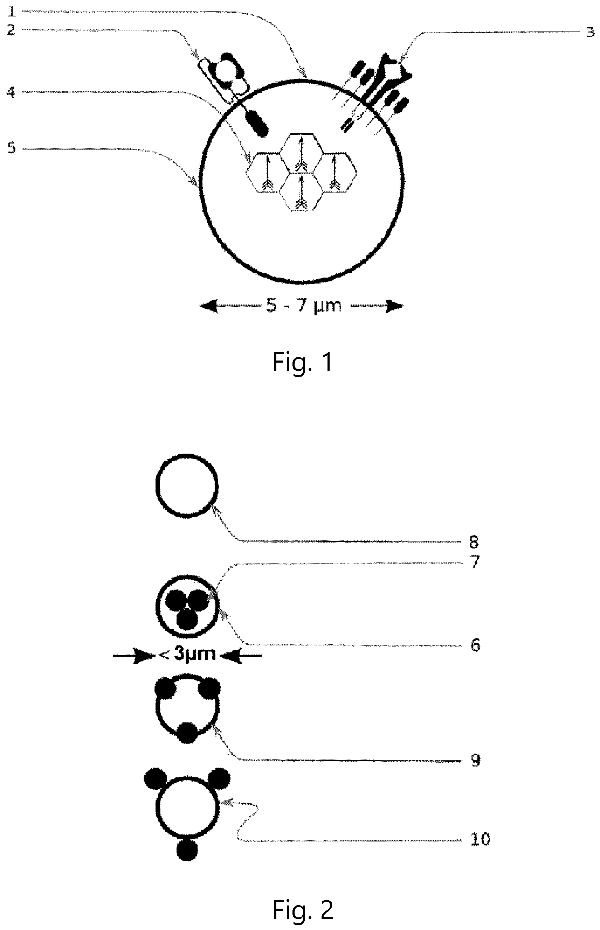

**Figure 1** shows cells 1, in particular genetically modified immunocompetent cells in the form of cultured $\gamma\delta$ CAR-T cells, e.g. v$\gamma$9v$\delta$2-T cells. The cells have incorporated into their cell membrane a co-stimulatory chimeric antigene receptor (CAR) 2 and a native $\gamma\delta$-T cell receptor ($\gamma\delta$-TCR) 3,. The cells can recognize the endogenous isopentenyl pyrophosphate (IPP) antigens on the cell surface of cancer cells.

The $\gamma\delta$ CAR-T cells used as an example in the present study are transfected after a selection to a specific type of patient-specific genetically modified cells and undergo a controlled cultivation phase.

At an early stage of this process, a suitable quantity of cells is removed and placed in a mixer not specifically shown in the figures, together with the desired substance, in this case nanoparticles and/or a cytotoxic agent.

A suitable quantity of microbubbles is added resulting in a mixture with a low viscosity. The mixture can be gently stirred to form a homogeneously mixed mixture (cell solution). All nanoparticles and/or cytotoxic substances that are useful for the planned therapy, e.g. stable isotopes and radioisotopes, isomers and so on, can be considered as nanoparticles and/or cytotoxic substances.

The microbubbles may be microbubbles with a lipid shell, which may be present in a phosphate-containing buffer solution.

Stirring must not generate any significant cavitation in the mixture, so that the perforation of the cell membranes and thus an increase in their permeability for the substance to be loaded does not start prematurely. A solution with a low viscosity is used for each component of the mixture in order to ensure a low overall viscosity of the mixture on the one hand and good adhesive contact with a reactor of a device for porating and loading cells on the other.

A desired low viscosity can be achieved, for example, by heating the mixture to 37° to 39° C, but preferably 37 °C. It is also possible to use aqueous alcohol solutions and/or other aqueous solutions of hydrocarbon-containing substances (e.g. an aqueous chloroform solution) in order to reduce the viscosity to a desired low value. When using such viscosity-reducing solutions, it is advantageous if the viscosity-reducing solutions are removed again after the cells have been porated and loaded, for example by evaporation or gentle centrifugation.

A typical quantity of cells at an early stage of the expansion process is in the range of $10^6$ to $10^8$.

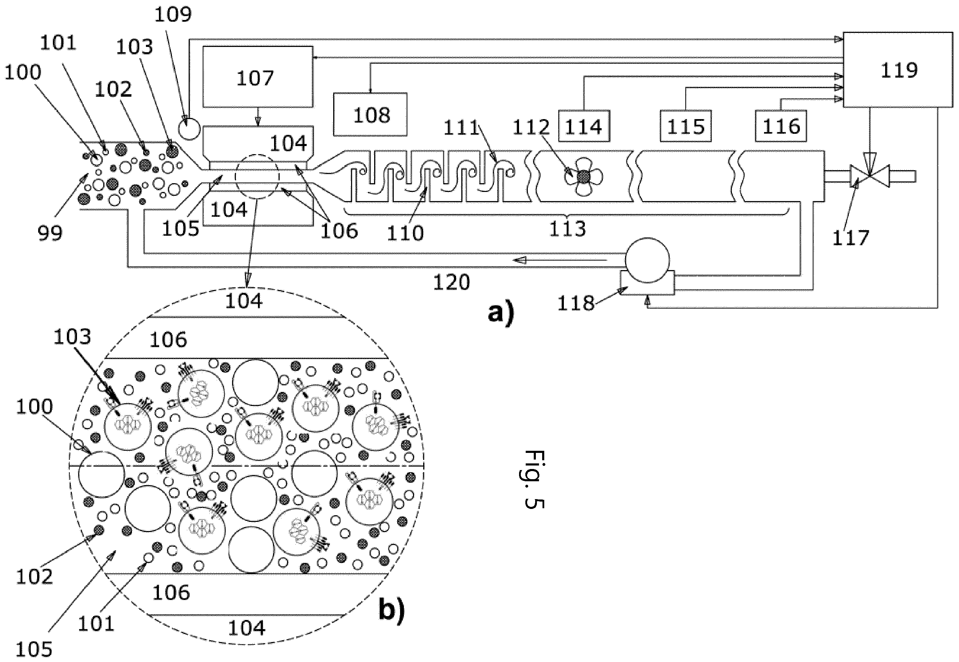

The following explanations refer to Figure 5a. **Figure 5a** shows a schematic representation of a preferred embodiment of a device for porating and loading cells according to the invention. The device comprises a collecting unit 99, a reactor with poration units 104 and an incubation section 113. The reactor also comprises an inlet and an outlet, both of which are not provided with a reference sign in the present case.

The mixed cell solution as described above is added in a suitable quantity to the collection unit 99 (e.g. in the form of a collection vessel) to collect the cells. The cells enter the reactor due to their own weight on the one hand and due to a capillary suction acting on them on the other. The reactor is hereinafter referred to as a sonoporation reactor, or "SORA" for short.

The SORA has a capillary section 105 in which the poration is carried out. On the one hand, this ensures that the cell solution is drawn into the SORA through a capillary organ. On the other hand, the capillary section 105 provides an optimum area of uniform sonoporation.

The SORA comprises two poration units 104 in the form of transducers, specifically ultrasonic transducers in the present case, which are arranged on the SORA in the present case. The ultrasonic transducers 104 are coupled to the SORA by means of an ultrasonic coupling medium 106.

The SORA, in particular the capillary section 105, has a round and/or oval cross-section in the present case, which can also be flattened in embodiments not specifically shown.

In the present case, the capillary section 105 is shown as a linear tube, but in embodiments not specifically shown, it can also have a meandering course.

The power required for the poration units 104 is provided by a generator amplifier unit 107 and recorded by a sensor 109. Signal evaluation and process control take place in real-time via a first control unit 119. The generator (amplifier) unit 107 may also be used to apply a stationary or non-stationary electric or electromagnetic field to the cell solution in the reactor respectively in the capillary section 105 and/or the incubation sector 113 for controlling permeabilization of the cell membrane.

After sonoporation and subsequent incubation, the $\gamma\delta$-CAR-T cells treated with this device showed a certain degree of uptake of the substance used, regardless of whether microbubbles were used or not. However, the use of microbubbles significantly increased the loading efficiency, as the permeability of the cell membranes could be increased due to micro-jets and/or microcavitation.

According to current theory of poration, it is assumed that reversible pores, i.e. pores that can be healed by the cell itself, are formed in the cell membranes, through which larger molecules can also pass.

Preferably, the poration arrangement preferably comprises several test and observation units 114, 115, and 116 along the incubation section 113.

In order to monitor and control the natural healing of the cell membranes, an electric or electromagnetic field is applied by transparent metal electrodes (vapor-deposited electrodes). The electrodes are placed on opposite walls of the poration arrangement, preferably in the section of the poration units and/or in the incubation section.

The electric or electromagnetic fields in the entire area of the SORA are preferably applied via the vapor-deposited transparent electrodes.

Preferably, the electrodes for low frequencies (up to approx. 1 MHz) are used on the inside of the reaction chamber and for higher frequencies (more than approx. 10 MHz) on the outside of the reaction chamber and are preferably vapour-deposited.

A second control unit 108 can be used to monitor electromagnetic fields that are applied along the poration arrangement by the transparent metal electrodes. The transparency of the electrodes is helpful in order to enable optimum operating conditions for the test and observation units 114, 115, and 116.

The increase in the permeability of the cell membranes caused by sonoporation heals within typically 40 to 60 minutes if the sonoporation does not reach the so-called "inertial area". In order to maintain a certain control over the size of the pores or membrane openings that are created according to current theory and their closing speed, the pores or membrane openings are mathematically modelled.

The state of the cell solution is shown schematically enlarged in **Figure 5b.** The $\gamma\delta$ CAR-T cells 103 are surrounded by empty microbubbles 100 or microbubbles coupled with nanoparticles and/or a cytotoxic substance, whereby the nanoparticles are in the form of stable isotopes 101, e.g. of boron or gadolinium, and the cytotoxic substance can be present in the form of radioisotopes 102, e.g. of technetium. The nanoparticles and/or the cytotoxic substance may also be present dissolved or as a suspension in the cell solution.

In order to be able to introduce the surrounding nanoparticles and/or the cytotoxic substance from the cell solution into the $\gamma\delta$ CAR-T cells, it is advantageous if the distance between the nanoparticles or the cytotoxic substance and the cells is manipulated by increasing the flow turbulence and the resulting pressure gradients. In the present example, this is achieved by means of baffles 110, which create vortices 111 in the incubation section 113. This area of the poration arrangement has a significantly larger cross-section than the SORA respectively the reactor, and, due to its shape and size, ensures that high Reynolds numbers, which ensure turbulent flow, are achieved despite the low flow velocity of the cell solution. Preferably, a large number of turbulence structures 110 define a meandering flow path through the incubation section 113. The flow rate of the cell solution can be kept constant by means of a flow adjustment element 112.

For optimum results, it is advantageous if the loading process is carried out gently and under constant observation via the test and observation units 114, 115, and 116. If the results are not satisfactory, i.e., if the loading efficiency

of the $\gamma\delta$ CAR-T cells is below a defined threshold value, the cell liquid is returned to the collection unit 99 by a pump 118 via a return line 120. If the test is satisfactory, the cell solution is removed via a valve 117.

The test and monitoring units 114, 115, and 116 are comparatively complex, which is why they are not described in detail here. In any case, it should be mentioned that they enable complete microscopic and radiometric monitoring of the cell healing process after poration, preferably in real-time. They can determine the relative number of successfully loaded $\gamma\delta$ CAR-T cells and the state of membrane permeabilization in real time and can evaluate these factors.

As an alternative to (ultra)sonic sonoporation, the use of laserporation, in particular laserporation using microbubbles, may be applied. One advantage of this process variant is the comparatively better controllability of process parameters, whereby the actual mechanisms of action on the cell solution and on the cells are similar to those described above.

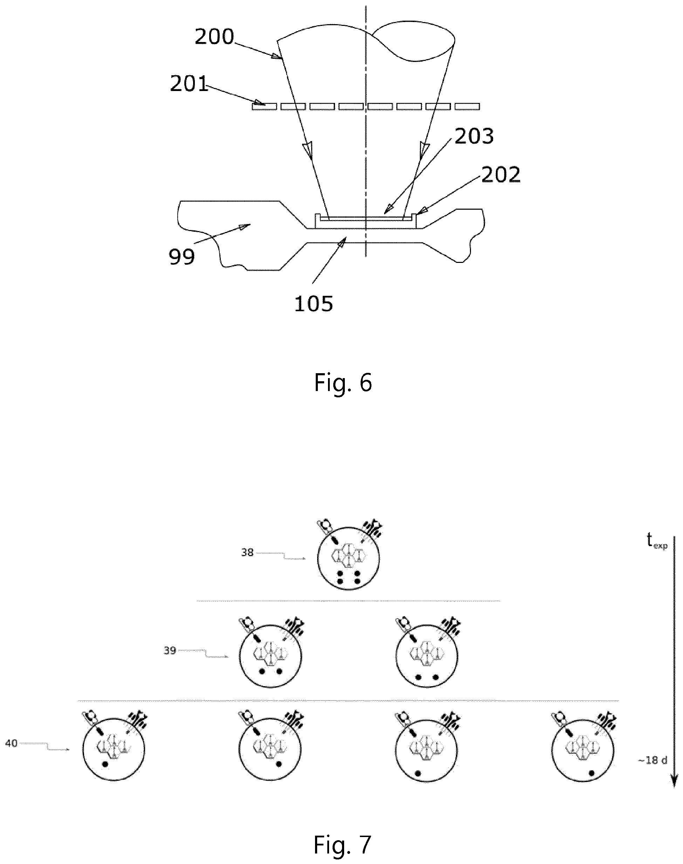

**Figure 6** shows a schematic view of a laser poration unit. A laser beam 200 is shaped in such a way that a flat intensity range ("top-hat") is present in the focus or is designed via diffraction gratings (transmissive or reflective) to match the geometry of the reactor. In the case of laserporation, the poration is created as a result of activation of the microbubbles by the laser on the flat bottom of a dish 202.

In the present case, the activation of the microbubbles refers to their ablation by the laser. The ablation of the microbubbles produces a so-called ablation recoil, which is directed through the dish 202 into the capillary section 105 of the reactor.

In the case of a PMMA dish 202 and an ArF type UV laser (193 nm), the ablation depth of the laser is approx. 2.0 $\mu$m to 2.3 $\mu$m per pulse at a fluence value of 100 mJ/cm$^2$ to 150 mJ/cm$^2$. If the resulting recoils are not strong enough, the material of the exchangeable dish 202 can be changed or the ablation surface of the dish 202 can advantageously be covered with a thin layer of liquid. The liquid layer should then advantageously provide at least partial transparency for the laser beam wavelength, so that as a result of the ablation, at the same point as described above, the inertial resistance of the overlying liquid layer (e.g. water) leads to an increase in the downward pressure surge. For optimum results, the layer thickness of the liquid should be measured and controlled in a non-contact and comparatively accurate manner (e.g. interferometrically or chromatically-confocal).

The nanoparticles and/or the cytotoxic substance can be added as a layer of free atoms or molecules, whereby they can also, as can be seen from **Figure 2,** be previously integrated in so-called microbubbles. If they are present as a layer of free atoms or molecules, a defined layer of empty microbubbles is added. The cells are located in a carrier liquid and a cell solution is formed by adding the nanoparticles and/or the cytotoxic substance.

The nanoparticles or the cytotoxic substance can be freely present as mentioned. They can also be present in an interior of the microbubbles (6), preferably chemically bound to each other or, for example, in the form of a chelate complex (7), in microbubble membranes, particularly mechanically embedded (9) or covalently bound to the microbubbles (10) from the outside. A preferred diameter of a microbubble is approx. 2 $\mu$m (preferably less than 3 $\mu$m). Preferably, the nanoparticles and/or the cytotoxic substance and the immunocompetent cells are added to the Petri dish in layers.

The reduced volume of the capillary section of the SORA compared to the rest of the poration arrangement ensures that all relevant parameters of the sonoporation are distributed homogeneously across the capillary section and that all cells in the capillary section are treated as equally as possible. This achieves a comparatively high efficiency of the loading process. The pressure P(t) applied by the ultrasonic transducer drives the nanoparticles, the cytotoxic substance and/or the loaded microbubbles into the vicinity of the $\gamma\delta$ CAR-T cells up to an optimum distance to the $\gamma\delta$ CAR-T cells. The microbubbles additionally open he cell membranes induced by their implosion due to the ultrasonic pressure and release their charge to the $\gamma\delta$ CAR-T cells.

P(t) and the duration $t_{total}$ of the exposure of the cell solution to ultrasonic pressure are partly based on empirical data derived from laboratory work, taking into account considerations of radioactive and biological activation. The exact process settings are specific to the microbubbles used and the specialized $\gamma\delta$-CAR-T cells to be used in a treatment.

The implosion of a microbubble, which is the process for delivering the payload, is determined by the Rayleigh-Plesset formula, which determines the change in the bubble radius R(t) over time:

$$R(t)\frac{d^2R(t)}{dt^2} + \frac{3}{2}\left(\frac{dR(t)}{dt}\right)^2 + \frac{4v_L}{R(t)}\frac{dR(t)}{dt} + \frac{2\gamma}{\rho_L R(t)} + \frac{\Delta P(t)}{\rho_L} = 0 \qquad (4)$$

where $\rho_L$ is the density of the surrounding liquid, $v_L$ is the kinematic viscosity of the surrounding liquid, $\gamma$ is the surface tension of the microbubble-liquid interface, $\Delta P(t) = P_\infty(t) - P_B(t)$ is the pressure difference between the uniform pressure inside the bubble $P_B(t)$ and $P_\infty(t)$ is the external pressure "infinitely" far from the bubble.

Formula 4 makes it possible to optimize the transducer's trigger signal in relation to the time of disintegration of a microbubble, whereby the free nanoparticles or the nanoparticles bound in microbubbles or the cytotoxic substance enter the T cells.

In order to be able to carry out at least partial permeabilization of the cell membranes of the $\gamma\delta$ CAR-T cells

used in the present example in a gentle and controlled manner, it is advantageous in the present embodiment if the following steps are carried out:

All components involved in the sonoporation process can be subjected to a precise tolerance control of the dimensions, as these significantly influence the intrinsic modes of the set-up. In particular, this requirement concerns the diameter of the microbubbles used, so that these are brought to a $\pm 10\%$ diameter tolerance by a micropore filter film before use.

Advantageously, the appliance settings are precisely maintained. Preferably, electrical or electromagnetic fields are used to control the reclosure of the cell membranes, for example, in a closed feedback loop.

P(t) in the formula (4) is periodic nature, $P(t') = P(t + t_{period})$. Even if a sinusoidal ultrasonic waveform gives good results, an optimized pulse profile shape with a constant period may be more favorable for optimal sonoporation.

Preferably, industrially produced and commercially available standard microbubbles are used for the poration procedure.

P(t) is optimized with respect to the amplitude $P_0$ (t), the period $t_{period}$ and the total duration of the sonoporation process ($t_{total}$). For each specific type of $\gamma\delta$ CAR-T cells cellular reactions such as cell membrane permeability and cytoskeletal fragmentation were investigated concerning their dependence on nuclear parameters such as the acoustic driving pressure P(t) and the distances between the emitting microbubbles.

For a given bubble diameter of ~ 2 $\mu$m and a cell with dimensions of a few $\mu$m, a two-dimensional simulation model according to formula (4) results in required pressure amplitudes of $P_0$ (t) ~ 400 hPa. A typical time for the endocytosis of the contents of the microbubbles into the immunocompetent cells is less than 1 $\mu$s. One aim of the simulations and design is to protect the carefully produced $\gamma\delta$ CAR-T cells from unnecessary stress and thus increase and optimize the effectiveness of the manufacturing process.

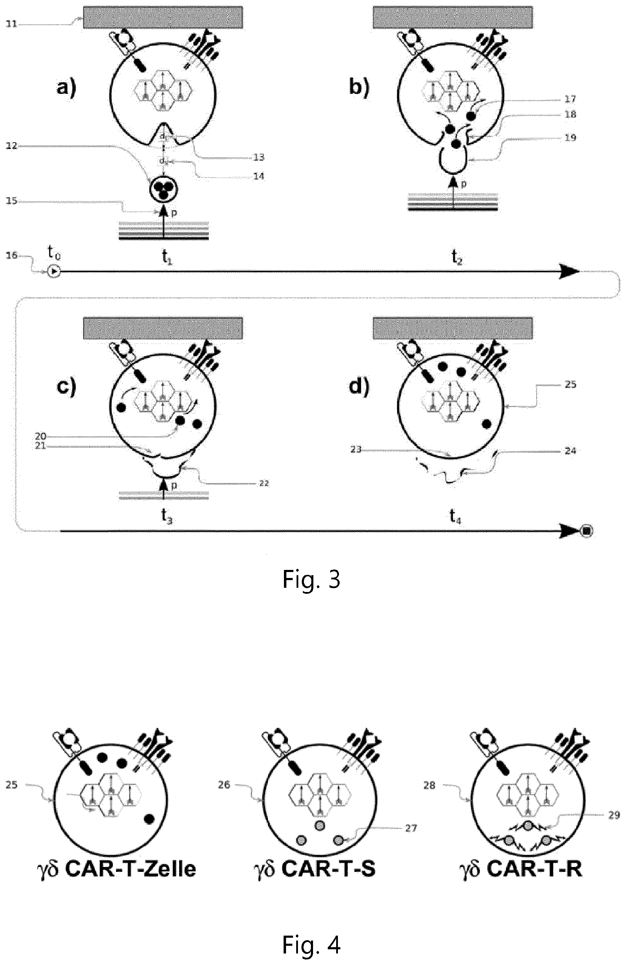

The sonoporation process, as can be seen in **Figure 3,** is divided into four phases $t_{1,2,3,4}$ after the ultrasonic transducer is switched on. After filling a sample holder, in this case an Opticell™ sample holder 11, with the corresponding number of $\gamma\delta$ CAR-T cells used in this case as genetically modified $\gamma\delta$ CAR-T cells, the ultrasonic transducer is switched on. The arrangement is reversed so that the free nanoparticles and/or the cytotoxic substance can diffuse from the loaded microbubbles 12 in the direction of the $\gamma\delta$ CAR-T cells (see **Figure 3a).** In the immediate vicinity of the microbubbles opposite the $\gamma\delta$ CAR-T cells, the application of ultrasound pressure via the ultrasound transducers leads to an indentation 13 with the depth dt of the cell membranes of the $\gamma\delta$ CAR-T cells. The size of dt depends on the distance $d_s$ between the microbubbles and the $\gamma\delta$ CAR-T cells, see reference sign 14. Both parameters dt and $d_s$ are functions of the applied pressure P(t), see reference sign 15, and thus implicitly of the time t, see reference sign 16.

The nanoparticles and/or the cytotoxic substance, see reference sign 17 in the **Figure 3b,** penetrate the $\gamma\delta$ CAR-T cell as soon as the sonoporation has led to an increase in the permeability of the cell membranes of the $\gamma\delta$ CAR-T cells, see reference sign 18. Then the microbubbles 12, see reference sign 19, disintegrate at the time $t_2$, typically in less than 1.0 $\mu$s after the start of exposure to P(t). Parallel to the disintegration of the microbubbles, the charge carried by the microbubbles 12 is transferred into the immunocompetent cells ($\gamma\delta$ CAR-T cells), for example by endocytosis, free diffusion and/or osmotic pressure.

This completes the loading process for the $\gamma\delta$ CAR-T cells. After the loading of the $\gamma\delta$ CAR-T cells, the nanoparticles and/or the cytotoxic substance are located inside the $\gamma\delta$ CAR-T cells, see reference sign 20 in **Figure 3c.** Thereafter, the cell membranes of the $\gamma\delta$ CAR-T cells close again, see reference sign 21, after the pressure has been reduced at time $t_3$. At this point, the disintegration of the microbubbles has progressed in such a way that they have disintegrated into smaller and no longer usable microbubbles, see reference sign 22.

**Figure 3d** illustrates the state of the $\gamma\delta$ CAR-T cells at the end of the loading process at time $t_4$, at which the cell membranes have closed again, see reference sign 23, and the microbubbles have completely disintegrated by implosion, as indicated by reference sign 24.

Residual microbubbles are filtered using standard molecular biological methods.

Ultrasonic pulses with a total duration of 20 s to 40 s are only applied in several cycles in order to estimate the achievable payload of the $\gamma\delta$ CAR-T cells under constant observation and to determine the actual own modes of the $\gamma\delta$ CAR-T cells.

The microbubbles themselves are provided in a phosphate buffer solution, for example. A total quantity of six million microbubbles per ml is considered the ideal concentration for the sonoporation process.

After a total number of 100,000 ultrasonic periods, a loading efficiency of the microbubbles of at least 10% is achievable if the sound pressure profile, the duration of the sound pressure periods, and the transducer geometry have been optimized.

It will be understood by the skilled person that the loading of the immunocompetent cells essentially depends on increasing the permeability of the cell membranes so that the nanoparticles which may be coupled to the microbubbles or the cytotoxic substance coupled to the microbubbles can enter the cell body of the immunocompetent cells. Therefore, the device according to the invention can also be used to perform electroporation or laserporation instead of sonoporation, whereby sonoporation is comparatively advantageous, as explained in detail above.

Loading depends on the radiological and biological activity of the patient-specific genetically modified cells in the patient, which is determined in advance for the treatment.

Preferably, the loading process takes place at an earlier stage of cell culture growth. Therefore, the intended payload per cell should not exceed the biologically acceptable, i.e. non-lethal, threshold. For the boron load, this would correspond to a payload of around 0.13 picograms [pg] per cell. This corresponds to $3 \times 10^{10}$ atoms of boron, for example, $^{11}$boron, $^{10}$boron or $^{nat}$boron, per cell.

In the case of radioisotopes, the dose exposure for the treated person should be taken into account. The patient-specific exposure is also influenced by factors such as the proliferation of $\gamma\delta$ CAR-T cells in a patient's body, in which they usually accumulate around the patient-specific genetically modified cells, for example, tumor cells.

The substance to be loaded can preferably be selected from one or more of the following four groups:

   a) Radioisotopes, e.g. $^{99m}$Tc, $^{177}$Lu, $^{18}$F or $^{211}$At, as a cytotoxic substance, whereby all other medically relevant radioisotopes may be also included in this group. On the one hand, these can be used in the first stage of radiological therapy without using an activation beam. Still, on the other hand, they can also be used in the second stage of radiological therapy without the use of an activation beam or for monitoring and checking the loaded cells before using the activation beam.

   b) Stable isotopes, e.g. $^{10}$B and $^{157}$Gd, which must first be activated by an activation beam of thermal or epithermal neutrons. They can be used in a second stage of radiological therapy.

   c) Naturally occurring elements - due to the special feature that, for example, the elemental boron is present as a composition of 19.9% $^{10}$B and 80.1% $^{11}$B, it is advantageous to use the cheaper naturally occurring variant, in this example $^{nat}$B. In addition, the example has the further advantage that $_{11}$B has an especially suitable cross-section for protons of around 1 barn at approx. 600 keV of proton energy. This means that $\gamma\delta$ CAR-T cells loaded with it are not only ideally suited for BNCT (boron-neutron capture therapy) but also for PBCT (proton-boron capture therapy).

   d) In addition to the above-mentioned radioisotopes or alternatively, one or more other cytotoxic substances.

The cells can also be loaded with several of the above-mentioned substances in parallel.

For a more straightforward description, the $\gamma\delta$ CAR-T cells loaded with the nanoparticles and/or the cytotoxic substance and used here are referred to as "T-ninjas". As illustrated in **Figure 4,** for a more precise differentiation, the T-ninjas are also divided into three subgroups according to the nature of the loaded nanoparticles and/or the loaded cytotoxic substance.

   a) T-ninja-R or $\gamma\delta$ CAR-T-R, see the reference sign (28), if the T-ninjas are loaded with a cytotoxic substance, in the present case radioisotopes (29) such as $^{99m}$Tc or $^{211}$At. By these radioisotopes an immediate radiologically therapy is of relevance for the first stage of a combined therapy against patient-specific altered cells. This comprises a cancer therapy without an activator beam, or, for example, a neutron beam or a proton beam.

   b) T-ninja-S or $\gamma\delta$ CAR-T-S, see the reference sign (26), if the T-ninjas are loaded with nanoparticles in the form of stable isotopes (27), such as $^{10}$B or $^{157}$Gd. For that cases, the radiologically relevant nuclear reaction against can be initiated at a desired point in time, for example in a combined therapy.

   c) T-ninja-SR or $\gamma\delta$ CAR-T-SR, which are not explicitly illustrated, as a combination of the T-ninja-R and T-ninja-S cells, whereby the combination of the cytotoxic substance present in the form of radioisotopes and nanoparticles present in the form of stable isotopes can be used particularly efficiently and flexibly.

In addition, all the T-ninjas described can also be used with additional substances at the same time, which will enable better monitoring and control of the manufacturing process and also the treatment of patients in certain phases of therapy.

Step 2 Distribution of nanoparticles and/or cytotoxic substance to daughter cells of loaded T-ninja cells after sonoporation

As can be seen in **Figure 7,** starting from the previously prepared T ninja cell culture, which was created as described above under optimal growth conditions, the payload of each T ninja cell in the cell culture is divided equally from the first generation 38 ($n_g$ = 1) to the respective subsequent generations 39 and 40 during the proliferation of the same. With each mitotic cell division, the individual cell payload of the daughter cells is reduced by half compared to the previous generation. The proliferation of the cells can take place over a period of up to three weeks. Constant monitoring of this proliferation phase ensures that the proliferation phase is terminated as soon as stagnation of cell division is

detected.

$$PL(n_g) = \left(\tfrac{1}{2} PL(1)\right)^{n_g} \qquad (5)$$

Clinical needs and ongoing monitoring will indicate the optimal timing and extent of cell proliferation and determine the final average payload of the T-ninja cells based on the Formula 5. In the case of radioisotopes, it is advantageous if the loading of the T-ninja cells is carried out immediately before the infusion of the T-ninja cells into the patient's body, as the T-ninja cells do not survive for long due to the ionizing particle radiation of the radioisotopes.

Overview of an embodiment of the invention from the cultivation and loading of immunocompetent cells to theranostic therapy against patient-specific genetically modified cells in the patient

The following description illustrates an example of the use of the method according to the invention and the device according to the invention for the provision of loaded immunocompetent cells in a theranostic therapy, in particular cancer therapy.

a) Due to the selectivity of a therapy following a successful loading of the cells, a histologic and/or a histopathological examination of the patient's affected tissue is carried out to determine, for example which specific cancer the patient has.

b) Preferably, allogeneic $\gamma\delta$ T-cells in sufficient numbers and quality are used according to the results of a), i.e. - to stay with the example - specifically for the respective cancer of the patient. The cells are transfected with a CAR gene that, among other things, codes for an scFv antibody domain mediating specificity for the respective cancer, whereby patient-specific $\gamma\delta$ CAR-T cells are obtained.

c) The patient-specific $\gamma\delta$ CAR-T cells transfected in this way are subjected to a cultivation phase while they are continuously monitored and controlled in order to select the right time for the next step of the process, namely the poration of the cell membranes and the loading of the cells with a substance.

d) The nanoparticles and/or the cytotoxic agent are preferably applied in layers or randomly mixed with the $\gamma\delta$ CAR-T cells and optionally mixed with a microbubble solution to obtain a cell solution, wherein the nanoparticles and/or the cytotoxic substance can be coupled with the microbubbles.

e) Sonoporation of the cell solution with predetermined parameters in the capillary section of a SORA of a device for porating and loading cells is performed, preferably under constant observation, with or without feedback control of the poration process. Alternatively, poration can also be achieved using laser poration. The poration results in a temporary permeabilization of the cell membranes of the $\gamma\delta$ CAR-T cells, which enables the uptake of the surrounding substance, in particular the nanoparticles and/or the cytotoxic substance.

f) For better control of the partial permeabilization of the cell membranes of the $\gamma\delta$ CAR-T cells, sonoporation is carried out in the presence of stationary or non-stationary electric or electromagnetic fields, whereby both sonoporation and the field control described here can be carried out with or without feedback control.

g) After sonoporation, the cell solution is swirled along an incubation section of the device under constant observation so that it can close the pores that have formed and, at the same time, absorb more of the surrounding material or more cells can absorb the surrounding material. By creating turbulence in the fluid flowing through the incubation section, the cell solution, the number of loaded cells can be increased due to an alternating effect of changing pressure gradients and, in particular, a favoring of natural diffusion. The cells can thus continue to absorb the surrounding substance under favorable conditions until they have closed their cell membranes again. This step can be carried out in the presence of an electrical or electromagnetic field control. If the field check determines that the loading efficiency is below a certain threshold value, the cell solution is fed back into the SORA until the loading efficiency has reached a satisfactory value. Using this procedure, a loading efficiency of up to 30% of the cells can be achieved, i.e. 30% of the porated cells are loaded with the desired substance after the poration and incubation steps.

h) The cell solution treated in this way is subjected to a further cultivation phase in which the number of loaded $\gamma\delta$

CAR-T cells is preferably increased by a factor of 1,000 to 100,000, whereby the load of a $\gamma\delta$ CAR-T cell is distributed evenly to the resulting daughter cells after cell division.

i) step g) and/or step h) is carried out under constant observation, whereby, for example, if radioisotopes which exhibit rapid $\beta$-decay, such as $^{18}$F, $^{99}$Tc or $^{177}$Lu, are used as the cytotoxic substance, the density and distribution of the loading in the cell solution can be determined without activation using an $n_{th}$ beam.

j) The correct time to administer the cells to a target area and/or the patient to be treated is determined.

k) The prepared cell solution is mixed with a preferably isotonic infusion solution and used to prepare an infusion for the patient to be treated.

**Claims**

1. Method for porating cells, preferably immunocompetent cells, for loading the cells with a substance, preferably with nuclides for radiotherapy, wherein the porating is performed in a porating device and wherein

   a. the cells to be porated are provided in a liquid together with the substance, and
   b. the membranes of the cells are permeabilized by sonoporation or laser poration, and
   c. the sonoporation or laser poration is performed in a reactor under real-time-monitoring.

2. Method according to claim 1, **characterized in that** the real-time monitoring is microscopic and/or radiometric monitoring.

3. Method according to claim 1 or claim 2, **characterized in that** the sonoporation or laser poration is performed in a capillary section (105) of the porating device.

4. Method according to one of the preceding claims, **characterized in that** after sonoporation or laser poration the cells are incubated in an incubation section (113) of the porating device, wherein preferably the incubation in the incubation section of the porating device is performed with gentle turbulence of the liquid.

5. Method according to claim 4, **characterized in that** the incubation in the incubation section (113) of the porating device is performed with application of an electric or electromagnetic field into the incubation section of the porating device.

6. Method according to claim 4 or claim 5, **characterized in that** the liquid with the cells from the incubation section is recirculated into the reactor of the porating device, in which repeated poration takes place.

7. Device for porating, in particular for sonoporation or laser poration, and for loading cells, in particular immunocompetent cells, in particular $\gamma\delta$ T cells, comprising:

   a. an inlet opening and an outlet opening for a cell solution with cells to be porated and at least one substance to be taken up by the cells as a load and preferably microbubbles, wherein a poration arrangement extends between the inlet opening and the outlet opening, wherein the poration arrangement has a collecting unit (99) arranged at the inlet opening for collecting the cell solution and a reactor in flow connection with the collecting unit, and wherein inside the reactor the cell membranes of the cells may be porated and the load may be uptaken by the cells, and
   b. at least one poration unit (104) arranged on the reactor, by means of which energy can be applied to the cell solution in such a way that the cell membranes of at least some of the cells are porated,

   wherein
   c. the reactor comprises a capillary section (105) in which the cells can be porated under real-time monitoring.

8. Device according to claim 7, **characterized in that** the poration arrangement comprises microscopic and/or radiometric means for real-time monitoring.

9. Device according to claim 7 or claim 8, **characterized in that** the capillary section (105) has an internal diameter or cross-section of 0.01 mm to 1 mm.

10. Device according to one of claims 7 to 9, **characterized in that** the capillary section (105) has a flattened form.

11. Device according to one of claims 7 to 10, **characterized in that** the poration arrangement comprises two poration units (104) arranged on the reactor, wherein the first poration unit being arranged on a first side of the reactor and the second poration unit being arranged on a second side of the reactor opposite the first side.

12. Device according to one of claims 7 to 11, **characterized in that** the at least one poration unit (104) is

a. a sound transducer, in particular an ultrasonic transducer, by means of which a sound pressure, in particular an ultrasonic pressure, can be generated from an electrical signal and directed onto the cell solution in the reactor, and/or
b. a laser by means of which a laser beam, in particular a top-hat laser beam with a top-hat intensity profile, can be generated and directed onto the cell solution in the reactor.

13. Device according to one of claims 7 to 12, **characterized in that** the poration arrangement comprises a tubular incubation section (113) between the reactor and the outlet opening, within which the porated cells can be incubated with the substance to be loaded for the purpose of enriching loaded cells, wherein preferably at least one control unit (108) is arranged at the incubation section (113), by means of which the quality and/or the quantity of the loaded cells can be controlled, wherein preferably the control unit is set up to detect ionizing particle radiation.

14. Device according to one of claims 7 to 13, **characterized in that** a return line (120) is arranged between the incubation section (113) and the outlet opening, within which a pump unit (118) is preferably arranged, wherein the return line ends in the collection unit.

15. Device according to one of claims 7 to 14, **characterized in that** a generator unit (107) is present, by means of which a stationary or non-stationary electric or electromagnetic field can be applied to the cell solution in the reactor and/or in the incubation sector (113) for controlling permeabilization of the cell membranes.

Fig. 1

Fig. 2

Fig. 3

γδ CAR-T-Zelle        γδ CAR-T-S        γδ CAR-T-R

Fig. 4

Fig. 5

Fig. 6

Fig. 7

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9855298 B2
- WO 2022241151 A2
- WO 2022238962 A1
- WO 2022241036 A1
- WO 2022147014 A2
- WO 2020109953 A1
- WO 2019181018 A1
- WO 9817342 A2
- CN 115215851 A
- CN 115232129 A
- FR 3120630 A1
- CN 112675442 A
- JP 2016159107 A
- JP 2018099059 A
- US 2010196983 A1
- US 2022062611 A1
- WO 2007028984 A1
- WO 2016005752 A1

**Non-patent literature cited in the description**

- **MIRZAEI et al.** *Prospects for chimeric antigen receptor (CAR) T cells: A potential game changer for adoptive T cell cancer immunotherapy,* 2016
- **MALOUFF et al.** *Boron Neutron Capture Therapy: A Review of Clinical Applications,* February 2021